# EUROPEAN PATENT APPLICATION

(11) **EP 4 534 113 A1**
(43) Date of publication of application: **09.04.2025**
(21) Application number: 23869285.9
(22) Date of filing: 05.09.2023
(51) Int. Cl.: A61L 9/12, G07F 11/50

(54) **DEVICE FOR COMBINING AND DIFFUSING MULTIPLE FRAGRANCES OR AROMAS AND THE LIKE**

(30) Priority: 08.08.2023 BR 102023015887
(71) Applicant: Noar Brasil Indústria e Comércio S.A., SP 09810-010 (BR)
(72) Inventor: GALVÃO, Claudia, 05440-000 São Paulo - SP (BR)
(74) Representative: Torner, Juncosa I Associats, SL
(86) International application number: PCT/BR2023/050293
(87) International publication number: WO 2025/030222

(57) **Abstract**

The present application for patent of invention refers to an equipment (1) for combining and diffusing multiple fragrances or aromas and the like. The highlight of the equipment (1) is that it comprises a mobile structure (13) with linear drive system (SL) and transmission mechanism (4), besides having an air supply system (SIA), to enable users to create customized combinations of fragrances from a variety of individual fragrance capsules (39). The equipment (1) was designed to prevent contamination between fragrances and assure a consistent and uniform release, proving olfactory experience to users, offering them an authentic olfactory experience, since it utilizes the concept of "dry smell". The invention that motivates the present application has its field of application focused on the perfumery industries, more particularly, destined for demonstration and experimentation with fragrances or aromas, and may also act in the field of beauty and well-being products.

## Description

### BRIEF PRESENTATION

The present application for patent of invention refers to an equipment for combining and diffusing multiple fragrances or aromas and the like. The highlight of the equipment comprises a mobile structure with linear drive system and transmission mechanism, besides having an air supply system, enabling users to create customized combinations of fragrances from a variety of capsules with individual fragrances. The equipment for combining and diffusing multiple fragrances or aromas, object of the present invention, was designed to prevent contamination between fragrances and assure a consistent and uniform release, offering an authentic olfactory experience to users, as it utilizes the concept of "dry smell".

### FIELD OF THE INVENTION

The invention that motivates the present application has its field of application focused on the perfumery industries, more particularly, destined for demonstration and experimentation with fragrances or aromas, and may also act in the field of beauty and well-being products.

### STATE OF THE ART

The current state of the art anticipates some patent documents related to equipment that provides fragrances and aromas, such as document BR 102019019346-8, published on March 3, 2021, and granted on June 6, 2023, which was filed by the very inventor. Specifically, the application granted refers to an electro-mechanical device for demonstrating multiple fragrances or invented aromas, which is nothing more than a compact e-catalog capable of offering the user the "dry" olfactory experimentation of different fragrances or aromas. The device in question is formed by a box with a movement mechanism that comprises a carousel, which houses up to one hundred cartridges containing fragrances or aromas, duly packaged in an absorbent cylindrical element. Upon being triggered via the application, the device causes the cartridge selected, housed in said carousel, to rotate thanks to an electric motor with gearbox, towards the nozzle connector. By means of a mechanical actuator, driven by electric motor, gearing and rack, the cartridge is pushed to the inside of the nozzle connector, at which time it receives a flow of air coming from a compressor, which upon traveling through a flexible hose, forces the elastomeric projections acting as one-way valve, whereby occupying the cartridge and, releasing only the gaseous portion of the fragrance to the top outlet hole of the cartridge *per se,* whereby enabling a trustworthy olfactory experimentation. Accordingly, when the cartridge is moved forward, its lid, which incorporates the one-way valve, moves the swivel handle which in turn releases the fragrance or aroma to the outlet hole. Once experimentation is complete, the cartridge returns to the initial position. At this point, the swivel handle also returns closing the outlet hole of the box and of the cartridge, which prevents the fragrances / aromas from mixing. Each fragrance or aroma is packaged in a cartridge (refill), and these cartridges disposed on a carousel, wherein each cartridge can offer over 30 bursts (experimentations). The cartridges can be replaced easily since they are encased on the carousel and accessible through a reload window on the outside of the shell.

However, the inventor in question noted the need to further the technological contribution and progression in the sector of experimenting with fragrances or aromas. But without creating objections to his application already granted, as referred to above.

At this stage, the inventor is ready to explain the differences contained in the patent application at hand, relative to the application BR 102019019346-8, already granted.

For example, in the present invention, the air supply occurs by means of two air blowers, with propellers, further having a set of flexible hoses connected in front and rear air supply devices, endowed with air inlet connectors, being interconnected in triangularly configured dispersing holes. In contrast, in application BR 102019019346-8, there is just a single flexible hose, which inflates air into the capsule by means of a single compressor.

In the present invention, there are three rotating drums, and may be comprised of three or more rotating drums, which are synchronized and aligned by rotation sensors and electro-magnets from an application, wherein said drums have hollow housings to provide for the insertion of a plurality of capsules. It is noted that the rotating drums enable the air inlet connectors from the air supply devices to enter into the capsules, either to inflate air, or to diffuse the fragrances mixed and selected by the user, by means of angled dispersing nozzles, which, for such, has an innovative linear drive system and transmission mechanism.

In contrast, application BR 102019019346-8 only has a 360° horizontal carousel, having a single nozzle connector linked to just one positioning sensor, which counts the turns of the axle of the electric motor, having a mechanical actuator formed by electric motor, a gearing that acts on a straight rack which, in turn, drives a drag stick that pushes and then retracts the cartridge, to the inside of said nozzle connector, jointly with the flow of air coming from a single flexible hose, from where the air inflated by a compressor enters into said cartridge, forcing the emission of the smell through the outlet hole of the lid of the box.

Moreover, the capsules in the application BR 102019019346-8, have a triangular prism shape, with closed bottom, having a valve. In the present invention, in contrast, the capsules have a rectangular prism shape, obtaining a hollow core, which enables the valve to be employed both at the front and rear part of the capsule, precisely so that the air supply system and linear drive system can operate, until the fragrances are diffused by said angled dispersing nozzles.

In other words, it is obvious that the present invention proposes an equipment for combining and diffusing multiple fragrances or aromas and the like, comprising significant advances over the application BR 102019019346-8 in terms of diffusion of "N" fragrances with improved quality. Since the application BR 1020190193468 only generates up to 100 (one hundred) types of fragrances or aromas, as in the operating mode.

Moreover, the state of the art anticipates certain patent documents related to equipment that provides fragrances and aromas, such as document US 5167877A entitled *"Air purifier with perfume dispensing control" -* used in the air purification sector, the equipment described releases different perfumes, through the passage of air coming from a pump, for the perfume cartridge previously selected and consequent departure of the perfume into the environment. It utilizes a solenoid air pump and electromagnet-driven carousel.

However, the prior art refers to an appliance that does not have a friendly interface with the user due to the lack of electronics, which enables commands by means of applications and control of the intensity and range of the perfume emission. Further, mechanically, the prior art presents constructive complexity, as it is made up of many components, besides requiring a considerably powerful solenoid pump, in light of the fact that the air is pumped into a liquid medium (perfume) to produce bubbles and thus carry the aroma to the outlet hole.

Provably, systems that utilize bubbling to drag the aroma do not promote a good olfactory experience. Moreover, mechanically, the prior art has the electromagnet-driven rotary table, whereas the return to the initial point is by means of springs. The mechanism is imprecise and is subject to wear and tear, for example, in the springs which when losing their elasticity, will increase the imprecision or will cause malfunctioning. Lastly, the constructive complexity and the excess of components may cause defects due to handling during demonstration, in addition to rendering its size rather bulky.

The document US 2004/0241053 A1, entitled *"Apparatus for dispensing volatile material into the environment"* - is used in the sector of scenting the air. The equipment consists of a set of elements, with perfume disposed on a rotary disk, such that upon positioning the chosen element, a flow of air generated by a blower passes through the element, carrying the perfume into the environment. The document cites a heater element of the perfumes to facilitate volatilization. The positioning system of the disk is electronic and controlled by sensors, motors and electronic circuits.

It is known that heating perfumes impairs them, since it releases more volatile notes in the first place. Another drawback is that there is an excess of mechanical components such as: gearings, axles, couplings, which result in an appliance of considerable volume, besides hampering the assembly and making the operation susceptible to defects or breakages of components.

The WO02/09776 A2, entitled *"System and methods for dispensing scents into the environment, and for providing scent-containing articles of manufacture" -* the equipment described belongs to the sector of dispensing multiple volatile products into the environment, including perfumes. A disk receives various cartridges containing the volatile substances. An electronic system that can be commanded by a computer, or directly in the equipment, positions the cartridge chosen and by means of heating and passage of air coming from the fan, the substance is released into the environment.

As in the preceding document, the use of heating to release the fragrances impairs the substances due to the premature evaporation of more volatile notes.

### GENERAL DESCRIPTION OF THE INVENTION

The present application for patent of invention refers to an equipment for combining and diffusing multiple fragrances or aromas and the like, which comprises a casing composed by a linear drive system, which is formed by a transmission mechanism comprised of a drive gear and drive gearings interconnected in two-way axles, wherein said two-way axles are connected on a U-shaped support, connected on a mobile structure endowed with a sliding element, which travels a linear guide, further having position sensors which delimit the forward and backward movement of said linear drive system.

The mobile structure of the equipment, further receives a triangular module of rotating drums, formed by a top left rotating drum, a top right rotating drum and a bottom rotating drum, being rotated by motors fastened on the rear wall of the mobile structure, besides having hollow housings to receive capsules storing multiple fragrances. The triangular module of rotating drums, in turn, is aligned and synchronized, by means of the communication of rotation sensors and electro-magnets, so that the capsules can receive inflated air from an air supply system. The air supply system is comprised of a rear air supply device, integrated to the U-shaped support, which is anchored on the mobile structure by means of guide axles, which pass through the rear wall and become fastened on the front wall of said mobile structure. Accordingly, the fragrances inflated in the capsules are dispersed by a front air supply device and diffused in angled dispersing nozzles.

### ADVANTAGES OF THE INVENTION

The present invention has the following advantages:
✔ It has rotating drums that enable the quick and easy change of the fragrance capsules, promoting the experimentation of a broad range of combinations of fragrances and aromas, and diverse possible sensory experiences;
✔ The functionality of pre-adjustment of combinations of fragrances in the computer system enables the olfactory experience to be personalized, making the equipment attractive for a broad range of users;
✔ By the capsules being designed to maintain the integrity and the potency of the fragrances during long periods of storage, reducing the frequency of replenishment and, therefore, the operating costs;
✔ It is an equipment that can be applied to a variety of environments, from private households to retail stores and public events;
✔ The possibility of integrating the system with a digital platform enables the collection of data and analyses of olfactory trends, providing valuable insights for developing new fragrances and product improvements;
✔ The air supply system incorporated in the equipment assures that the distribution of the fragrances is carried out in consistent and uniform manner, maximizing the efficacy of each capsule included by the fragrances, besides guaranteeing an authentic olfactory experience;
✔ By the linear drive of the equipment providing a precise control over the amount of fragrance released, enabling a finer personalization of the olfactory experience, in addition to reducing fragrance wastage;
✔ By the integrated drive mechanism, guaranteeing the smooth and efficient movement of the fragrance capsules inside the equipment, reducing the mechanical wear and tear and extending the useful life of the equipment;
✔ It has the capacity to combine countless fragrances, opening up an almost infinite spectrum of possibilities for the creation of personalized perfumes, giving the users the freedom to experiment and create its own exclusive mixtures;
✔ The fragrance capsule and the air supply system prevent contamination between different fragrances, assuring that each perfume created is pure and not adulterated by residues of prior fragrances;
✔ The air supply, jointly with the linear drive, enables the release of fragrances without the use of additional chemical products or solvents, being safer for the user and more ecological.

### DESCRIPTION OF THE DRAWINGS

In order to assist with understanding, the following figures are appended:

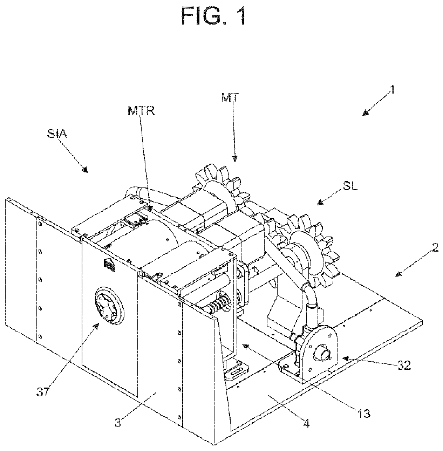

Fig. 1: shows the perspective view of the equipment for combining and diffusing multiple fragrances or aromas and the like;

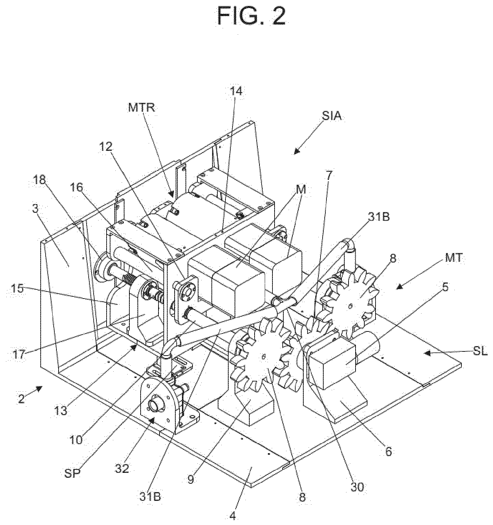

Fig. 2: shows the upside down perspective view of the equipment for combining and diffusing multiple fragrances or aromas and the like;

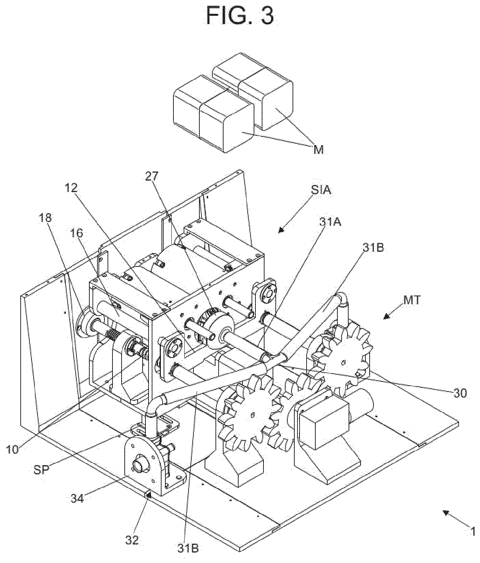

Fig. 3: shows the perspective view of the equipment for combining and diffusing multiple fragrances or aromas and the like, displaying the blown-up motors, showing the rear air flute;

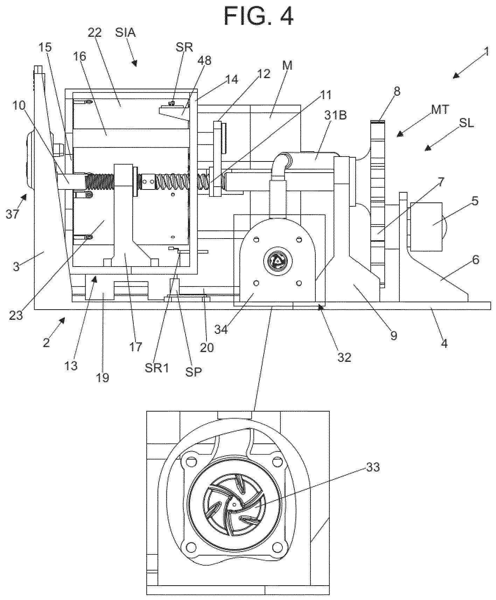

Fig. 4: shows the side view of the equipment for combining and diffusing multiple fragrances or aromas and the like, showing the blown-up detail of the air blower;

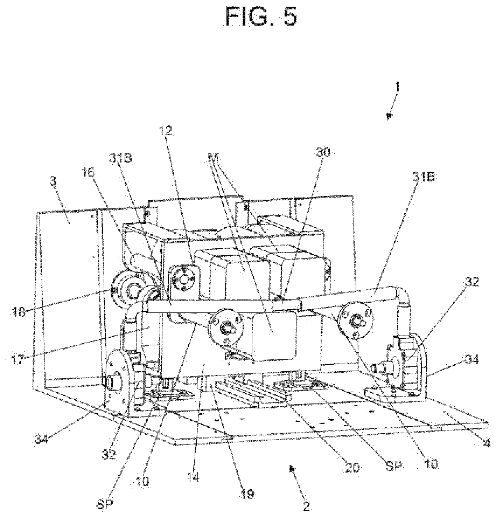

Fig. 5: shows the perspective view of the equipment for combining and diffusing multiple fragrances or aromas and the like, showing the sliding element and the linear guide;

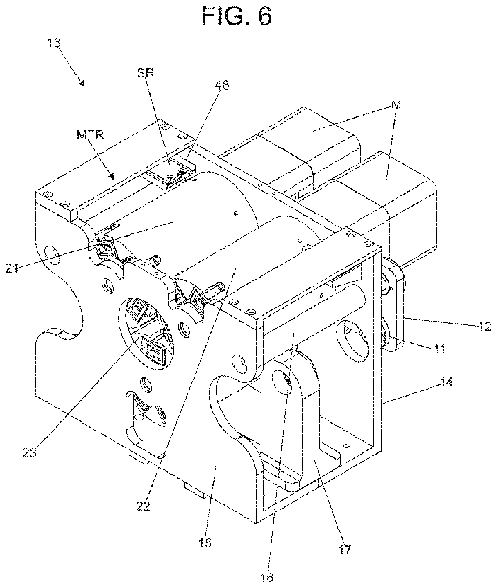

Fig. 6: shows the perspective view of the equipment for combining and diffusing multiple fragrances or aromas and the like, showing the mobile structure only;

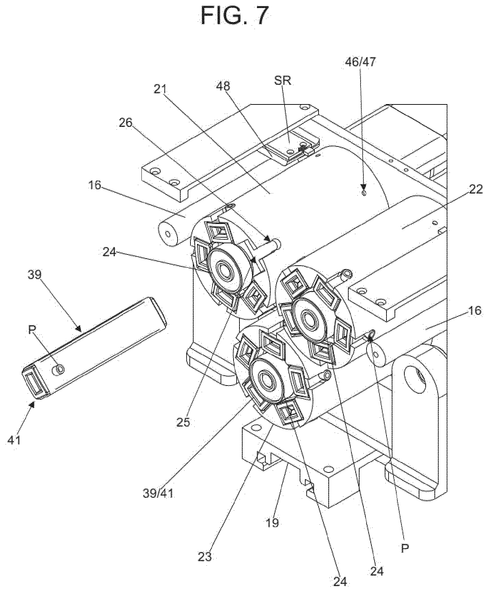

Fig. 7: shows the perspective view of the equipment for combining and diffusing multiple fragrances or aromas and the like, showing the module of rotating drums, with a blown-up capsule;

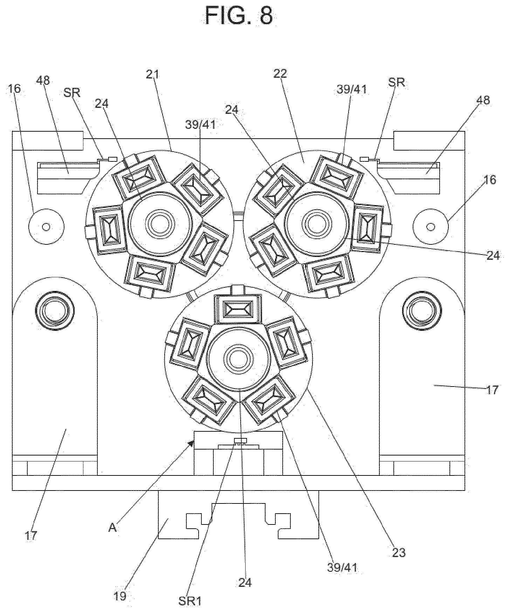

Fig. 8: shows the front view of the equipment for combining and diffusing multiple fragrances or aromas and the like, showing the module of rotating drums;

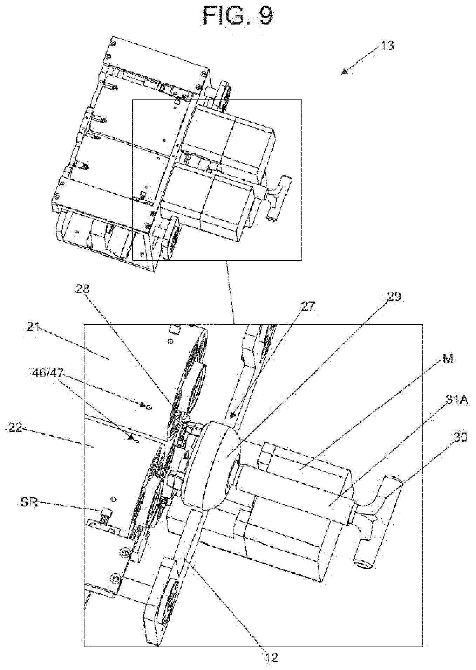

Fig. 9: shows the perspective view of the equipment for combining and diffusing multiple fragrances or aromas and the like, showing the blown-up detail of the rear air flute of the air supply system;

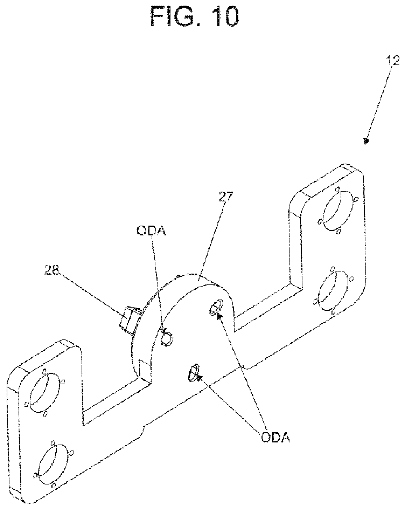

Fig. 10: shows the perspective view of the equipment for combining and diffusing multiple fragrances or aromas and the like, showing the blown-up detail of the prior air flute and the angled dispersing nozzles;

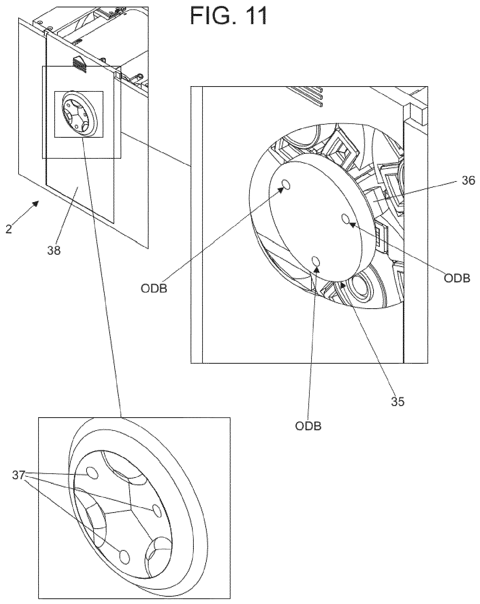

Fig. 11: shows the perspective view of the equipment for combining and diffusing multiple fragrances or aromas and the like, showing the blown-up detail of the rear air flute of the air supply system, entering the rotating drums;

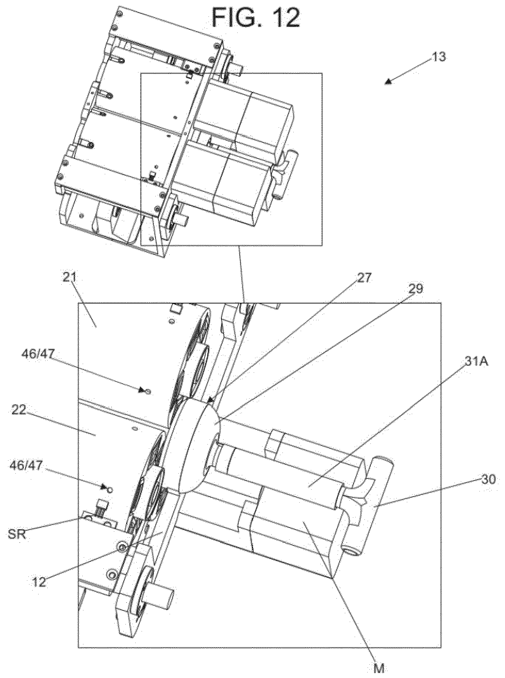

Fig. 12: shows the perspective view of the assembled capsule;

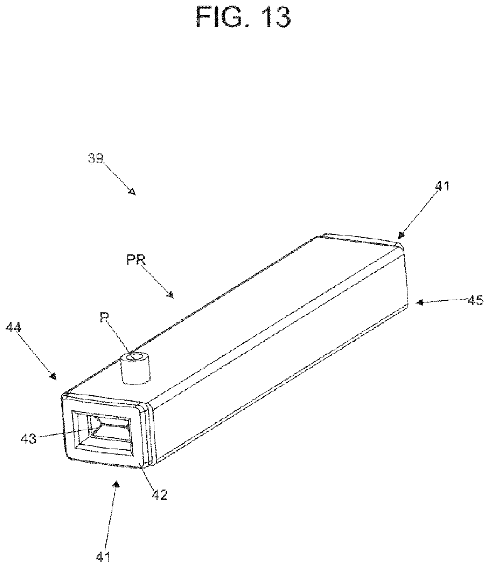

Fig. 13: shows the perspective view of the blown-up assembled capsule;

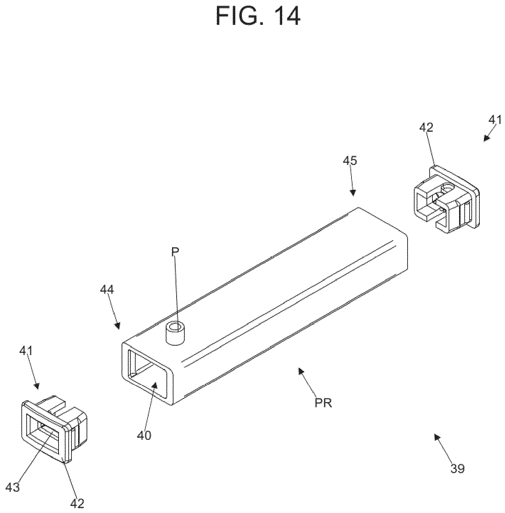

Fig. 14: shows the view of the rotating drums;

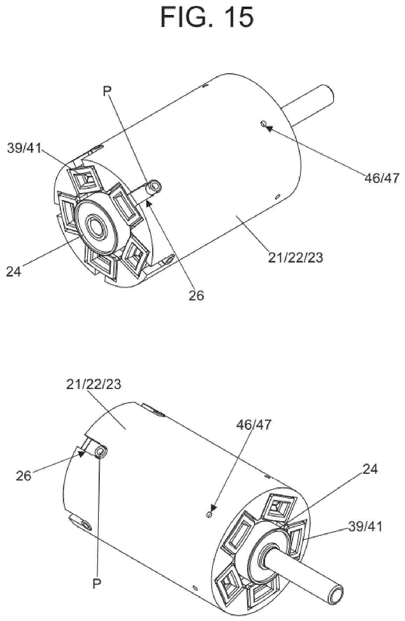

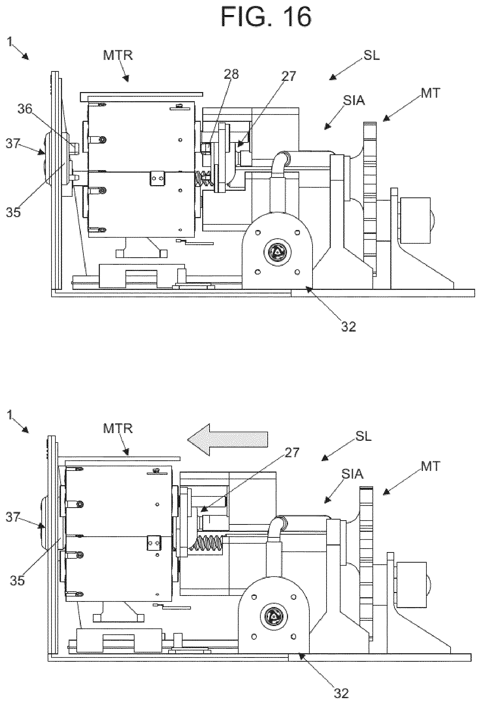

Fig. 15: shows the schematic view of the equipment for combining and diffusing multiple fragrances or aromas and the like, showing the linear drive system in use.

### DETAILED DESCRIPTION OF THE INVENTION

The EQUIPMENT FOR COMBINING AND DIFFUSING MULTIPLE FRAGRANCES OR AROMAS AND THE LIKE, object of this application for patent of invention, consists of an equipment (1) for combining and diffusing multiple fragrances or aromas and the like. The system (1) includes a casing (2) comprised of a front wall (3) and bottom base (4), the casing (2) of which includes a linear drive system (SL), which is formed by a transmission mechanism (TM) configured by an electric motor (5) supported on a bearing (6), whose electric motor (5) drives a motor gear (7), which is interspersed with drive gearings (8). Both drive gearings (8) are anchored on bearing supports (9), being interconnected in two-way axles (10), which actuate and pass through threaded flanges (11) situated in a U-shaped support (12), which is fastened on a mobile structure (13).

The U-shaped support (12) is incorporated by a rear air supply device (27), being integrated to an air supply system (SIA). In turn, the U-shaped support (12) is anchored on the mobile structure (13) by means of guide axles (16), which pass through the rear wall (14) and are fastened on the front wall (15) of the mobile structure (13).

Thereafter, said two-way axles (10) pass through the rear wall (14) of the mobile structure (13), being supported on threaded flange bearings (17), which are fastened at the centered ends of the mobile structure (13), whereas the two-way axles (10) pass through the front wall (15) of said mobile structure (13), then being connected on roll bearing flanges (18), which are fastened on the front wall (3) of the casing (2).

The mobile structure (13) is anchored on a sliding element (19), which travels a linear guide (20) fastened on the lower base (4) of the casing (2), enabling it to execute a forward and backward movement. In turn, the bottom base (4) accommodates two position sensors (SP), which are beneath the mobile structure (13), in order to detect the forward and backward position of said mobile structure (13), upon actuation of the linear drive system (SL).

Incidentally, the rear wall (14) of the mobile structure (13) receives the fastening of motors (M), which are responsible for rotating the triangular module of rotating drums (MRD), more precisely being formed by a top left rotating drum (21), a top right rotating drum (22) and a bottom rotating drum (23), which are fastened by means of rolled axles (24), both on the rear wall (14), as on the front wall (15) of the mobile structure (13). Incidentally, both the top left rotating drum (21), and the top right rotating drum (22) and the bottom rotating drum (23), which configure the module of rotating drums (MRD), are comprised of a plurality of hollow housings (25) and front recesses (26).

Additionally, the top left rotating drum (21), the top right rotating drum (22) and the bottom rotating drum (23), have cylindrical seats (46), which accommodate electro-magnets (47) that communicate with rotation sensors (RS) supported on supports (48) fastened on the top part of the mobile structure (13), having Further, a rotation sensor (RS1) included in a bottom opening (A) of the rear wall (14) of the mobile structure (13), being located beneath the bottom rotating drum (23), for communicating with the rotation sensor (RS1) included in the bottom opening (A).

The air supply system (SIA) is formed by the rear air supply device (27) incorporated on the U-shaped support (12), being comprised of connector ducts (28) interconnected in triangularly configured dispersing holes (ODA). Further, the rear air supply device (27) receives the coupling of an air inlet connector (29), which is coupled by a transverse flexible hose (31A), connected on a forked joining element (30). In turn, the forked joining element (30) is connected in two transverse flexible hoses (31B), being interconnected on air blowers (32) endowed with propellers (33), being fastened on supporting elements (34) supported on the lower base (4) of the casing (2).

The air supply system (SIA) is also composed of a rear air supply device (35), which comprises connector ducts (36) interconnected in triangularly configured dispersing holes (ODB), being fastened opposite angled dispersing nozzles (37), in a closing profile (38) of the casing (2).

The equipment (1) for combining and demonstrating multiple fragrances or aromas and the like, is also configured by capsules (39) being configured by a rectangular prismatic body (RP) endowed with an internal hollow channel (40), such that the internal hollow channels (40) receive flexible one-way valves (41) endowed with elastomeric headers (42), which comprise converging flexible vanes (43), both at the front end (44) and at the end rear (45). Accordingly, the capsules (39) may store "N" types of fragrances or aromas.

Moreover, the capsules (39) are introduced into hollow housings (25) of the top left rotating drum (21), of the top right rotating drum (22) and of the bottom rotating drum (23), its displacement course having limited in the hollow housings (25), by means of its projections (P), which access the front recesses (26) of the top left rotating drum (21), of the top right rotating drum (22) and of the bottom rotating drum (23), besides assisting with the change of said capsules (39). Afterwards, the flexible one-way valves (41) are incorporated both at the front end (44) and at the end rear (45) of the rectangular prism body (PR) of the capsules (39), facing towards the connector ducts (28) interconnected in triangularly configured dispersing holes (ODA) of the rear air supply device (27), and towards the connector ducts (36) interconnected in triangularly configured dispersing holes (ODB) of the air supply device anterior (35).

### FUNCTIONING

The equipment (1) connects to a computer system, wired or wireless, commanded by means of an application, being capable of locating and combining the desired fragrances, which are stored in the capsules (39) inserted in the hollow housings (25) of the top left rotating drum (21), of the top right rotating drum (22) and of the bottom rotating drum (23).

Accordingly, the user selects and combines the desired fragrances by means of the computer system, commanded by an application. Then, after selecting and combining the fragrances by the user in the computer system, the rotation sensors (RS) and the rotation sensor (RS1) of the triangular module of rotating drums (MRD), by means of the communication with the electro-magnets (47) and the motors (M), synchronize the top left rotating drum (21), the top right rotating drum (22) and the bottom rotating drum (23) causing the capsules (39) with the fragrances selected by the user to align with the connector ducts (28) interconnected in triangularly configured dispersing holes (ODA) of the rear air supply device (27) and, with the connector ducts (36) interconnected in triangularly configured dispersing holes (ODB) of the air supply device anterior (35).

Consequently, the transmission mechanism (TM) of the linear drive system (SL) is driven, enabling the mobile structure (13), which houses the triangular module of rotating drums (MRD), be recoiled by means of the two-way axles (10) of the linear drive system (SL), until the connector ducts (28) interconnected in triangularly configured dispersing holes (ODA) of the rear air supply device (27) enter into the flexible one-way valves (41) incorporated at the rear ends (45) of the capsules (39), included in the hollow housings (25) of the top left rotating drum (21), the top right rotating drum (22) and the bottom rotating drum (23), whose recoil limit of the mobile structure (13) of the linear drive system (SL), is given by the position sensors (PS).

Moreover, when the connector ducts (28) enter into the flexible one-way valves (41) situated at the rear ends (45) of the capsules (39), causing the opening of the converging flexible vanes (43) of the capsules (39) selected, the air supply system (SIA) inflates the air by means of its air blowers (32) and of its propellers (33), inside the capsules (39) with the fragrances selected by the user in the computer system.

Once the air supply in the capsules (39) has been selected, the transmission mechanism (TM) of the linear drive system (SL) is again driven, enabling the mobile structure (13), to be advanced by means of the two-way axles (10) of the linear drive system (SL), whose forward limit of the mobile structure (13), is given by the position sensors (SP). Then, until the connector ducts (36) interconnected in triangularly configured dispersing holes (ODB) of the air supply device anterior (35), enter into the flexible one-way valves (41) incorporated into the front ends (44) of the capsules (39) selected, there is a burst of emission of the smell of the mixture of the fragrances chosen by the user, by means of the angled dispersing nozzles (37), providing the user an authentic olfactory experience, since it utilizes the concept of "dry smell". This occurs because the exhalation system has no heating process, preventing the fragrance from being impaired by evaporation of the more volatile notes. When the user wishes to try a new fragrance or aroma, he or she simply selects it in the application and the appliance proceeds with the selection of the new fragrance chosen.

## Claims

1. An EQUIPMENT FOR COMBINING AND DIFFUSING MULTIPLE FRAGRANCES OR AROMAS AND THE LIKE consists of an equipment (1) for combining and demonstrating multiple fragrances or aromas, comprised of a casing (2), equipped with an electric motor (5) supported on a bearing (6), further having flexible hoses and capsules (39) endowed with flexible one-way valves (41), which comprise elastomeric headers (42) and converging flexible vanes (43), further having projections (P), whose equipment (1) wherein said equipment has in the casing (2) a linear drive system (SL), formed by a transmission mechanism (TM) comprised of a drive gear (7) and drive gearings (8) interconnected on two-way axles (10), wherein the two-way axles (10) are connected on a U-shaped support (12), connected in a mobile structure (13) endowed with a sliding element (19), which travels a linear guide (20), further having position sensors (SP) that delimit the forward and backward movement of the linear drive system (SIA); by the mobile structure (13) receiving a triangular module of rotating drums (MRD), formed by a top left rotating drum (21), a top right rotating drum (22) and a bottom rotating drum (23), being rotated by motors (M) fastened on the rear wall (14) of the mobile structure (13), besides having hollow housings (25) for receiving capsules (39) storing multiple fragrances; further, by the triangular module of rotating drums (MRD) being aligned and synchronized, by means of the communication of rotation sensors (RS), rotation sensor (RS1) and electro-magnets (47), so that the capsules (39) can receive inflated air from an air supply system (SIA), comprised of a rear air supply device (27), integrated to a U-shaped support (12), anchored on the mobile structure (13) by means of guide axles (16), which pass through the rear wall (14) and are fastened on the front wall (15) of said mobile structure (13); further, by the fragrances inflated in the capsules (39) being dispersed by a prior air supply device (35) and diffused in angled dispersing nozzles (37).

2. The EQUIPMENT FOR COMBINING AND DIFFUSING MULTIPLE FRAGRANCES OR AROMAS AND THE LIKE according to claim 1, wherein the transmission mechanism (TM) of the linear drive system (SL), is configured by an electric motor (5) supported on a bearing (6), whose electric motor (5) drives a drive gear (7), which is interspersed by drive gearings (8); by the drive gearings (8) being anchored on bearing supports (9) and interconnected on two-way axles (10), which actuate and pass through threaded flanges (11) situated on the U-shaped support (12) fastened on a mobile structure (13).

3. The EQUIPMENT FOR COMBINING AND DIFFUSING MULTIPLE FRAGRANCES OR AROMAS AND THE LIKE according to claims 1, wherein the two-way axles (10) pass by the rear wall (14) of the mobile structure (13) is supported on threaded flange bearings (17), which are fastened on the centered ends of the mobile structure (13), whereas, the two-way axles (10) pass through the front wall (15) of said mobile structure (13), and then connected on roll bearing flanges (18), which are fastened on the front wall (3) of the casing (2).

4. The EQUIPMENT FOR COMBINING AND DIFFUSING MULTIPLE FRAGRANCES OR AROMAS AND THE LIKE according to claim 1, wherein the top left rotating drum (21), the top right rotating drum (22) and the bottom rotating drum (23) comprise front recesses (26) to promote the limitation and encasement and removal of the capsules (39) by means of its projections (P) lodged in their hollow housings (25).

5. The EQUIPMENT FOR COMBINING AND DIFFUSING MULTIPLE FRAGRANCES OR AROMAS AND THE LIKE according to claims 1 and 4, wherein the top left rotating drum (21), the top right rotating drum (22) and the bottom rotating drum (23) have cylindrical seats (46) which accommodate electro-magnets (47) that communicate with rotation sensors (RS) supported on supports (48) fastened on the top part of the mobile structure (13), further having a rotation sensor (RS1) included in a bottom opening (A) of the rear wall (14) of the mobile structure (13), being located beneath the bottom rotating drum (23).

6. The EQUIPMENT FOR COMBINING AND DIFFUSING MULTIPLE FRAGRANCES OR AROMAS AND THE LIKE according to claim 1, wherein the rear air supply device (27) of the air supply system (SIA) is comprised of connector ducts (28) interconnected in triangularly configured dispersing holes (ODA); further, by the rear air supply device (27) receiving the coupling of an air inlet connector (29), which is equipped with a flexible hose transversal (31A), encase into forked joining element (30); by the forked joining element (30), in turn, being connected on two transverse flexible hoses (31B), which are encased into air blowers (32) endowed with propellers (33), being fastened on supporting elements (34) supported on the lower base (4) of the casing (2).

7. The EQUIPMENT FOR COMBINING AND DIFFUSING MULTIPLE FRAGRANCES OR AROMAS AND THE LIKE according to claims 1 and 6, wherein the air supply system (SIA) is composed of a rear air supply device (35), which comprises connector ducts (36) interconnected in triangularly configured dispersing holes (ODB), being fastened opposite angled dispersing nozzles (37), in a closing profile (38) of the casing (2).

8. The EQUIPMENT FOR COMBINING AND DIFFUSING MULTIPLE FRAGRANCES OR AROMAS AND THE LIKE according to claim 1, wherein the capsules (39) is configured by a rectangular prismatic (RP) body endowed with an internal hollow channel (40), for receiving the flexible one-way valves (41), both at the front end (44) and at the rear end (45) of the rectangular prismatic (RPI) body, such that the capsules (39) upon being included in the hollow housings (25) of the top left rotating drum (21), of the top right rotating drum (22) and of the bottom rotating drum (23), enable the flexible one-way valves (41) to face both the connector ducts (28) interconnected in triangularly configured dispersing holes (ODA) of the rear air supply device (27), and for the connector ducts (36) interconnected on triangularly configured dispersing holes (ODB) of the air supply device anterior (35).

9. The EQUIPMENT FOR COMBINING AND DIFFUSING MULTIPLE FRAGRANCES OR AROMAS AND THE LIKE according to claim 1, wherein the rotation sensors (RS) and the rotation sensor (RS1) of the triangular module of rotate drums (MRD), by means of the communication with the electro-magnets (47) and the motors (M), synchronize the top left rotating drum (21), the top right rotating drum (22) and the bottom rotating drum (23) causing the capsules (39) with the fragrances selected by the user to align with the connector ducts (28) interconnected in triangularly configured dispersing holes (ODA) of the rear air supply device (27) and, with the connector ducts (36) interconnected in triangularly configured dispersing holes (ODB) of the air supply device anterior (35).

10. The EQUIPMENT FOR COMBINING AND DIFFUSING MULTIPLE FRAGRANCES OR AROMAS AND THE LIKE according to claim 1, wherein the mobile structure (13) of the linear drive system (SL) is recessed to enable the connector ducts (28) interconnected in triangularly configured dispersing holes (ODA) of the rear air supply device (27) enter into the flexible one-way valves (41) incorporated at the rear ends (45) of the capsules (39), included in the hollow housings (25) of the top left rotating drum (21), the top right rotating drum (22) and the bottom rotating drum (23).

11. The EQUIPMENT FOR COMBINING AND DIFFUSING MULTIPLE FRAGRANCES OR AROMAS AND THE LIKE according to claim 1, wherein the mobile structure (13) of the linear drive system (SL) is advanced by means of the two-way axles (10) of the linear drive system (SL), causing the connector ducts (36) interconnected in triangularly configured dispersing holes (ODB) of the air supply device anterior (35) to enter into the flexible one-way valves (41) incorporated at the front ends (44) of the capsules (39) selected, carrying out a dispersion and the diffusion of the smell of the mixture of the fragrances chosen by the user, by means of the angled dispersing nozzles (37).
